Europäisches Patentamt

European Patent Office

Office européen des brevets

(11) Publication number: **0 019 877**
**B1**

(19)

# (12) EUROPEAN PATENT SPECIFICATION

(45) Date of publication of patent specification: **29.04.87**

(51) Int. Cl.⁴: **C 12 N 15/00**

(21) Application number: **80102888.7**

(22) Date of filing: **23.05.80**

(54) **Method of preparing a microorganism, harboring a plasmid, with stabilized characteristics, and microorganism obtained in this manner.**

(30) Priority: **23.05.79 JP 63467/79**

(43) Date of publication of application:
**10.12.80 Bulletin 80/25**

(45) Publication of the grant of the patent:
**29.04.87 Bulletin 87/18**

(84) Designated Contracting States:
**CH DE FR GB LI**

(56) References cited:

NATURE, vol. 261, June 3, 1976, C WEISSMAN et al.: "Reduction of possiblehazards in the preparation of recombinant plasmid DNA", pages 428-429

CURR. TOP. MICROBIOL. IMMUNOL., vol. 78, 1977. J. COLLINS: "Gene cloning with small plasmids", pages 121-170

The file contains technical information submitted after the application was filed and not included in this specification

(73) Proprietor: **AJINOMOTO CO., INC.**
**5-8, Kyobashi 1-chome, Chuo-ku**
**Tokyo 104 (JP)**

(72) Inventor: **Miwa, Kiyoshi**
**531, Iwase**
**Matsudo-shi, Chiba-ken (JP)**
Inventor: **Momose, Haruo**
**2-24-2, Tamanawa**
**Kamakura-shi, Kanagawa-ken (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

Courier Press, Leamington Spa, England.

## Description

This invention relates to a method for stabilizing the characteristics of a microorganism harboring a plasmid (plasmid-containing microorganisms).

There were some attempts to construct useful microorganisms by transforming Escherichia strains using a plasmid which is inserted with a valuable genetic information.

However, in general, the plasmid is apt to be eliminated during the culture, and hence the microbial characteristics controlled by the plasmid become unstable in the microbial cell population. This phenomenon restricts the application of the plasmid-containing microorganisms for commercial purposes.

Such instability can be diminished by conditions under which only the plasmid-containing cells survive and the plasmid-free cells, formed by the above-mentioned phenomenon, are selectively killed.

According to a method based on this principle a plasmid with an appropriate gene or genes relating to antibiotic resistance is used and a microorganism harboring the plasmid is cultured in a medium containing the antibiotic(s), so that only the plasmid-comprising cells can survive owing to the antibiotic resistance, and the plasmid-free cells are killed because of their sensitivity to the antibiotic(s). In this method, however, large amounts of antibiotic(s) are required and, therefore, it is hardly applicable for industrial purposes.

The object underlying the present invention is therefore to provide a method by which the genetic characteristics of a transformant microorganism of the genus Escherichia, which harbours a plasmid, may be effectively stabilized.

The method for stabilizing the characteristics of a transformant microorganism of the genus Escherichia harboring a plasmid is characterized in that a plasmid obtained from a microorganism of the genus Escherichia, said plasmid being inserted with a chromosomal DNA fragment controlling the independence of streptomycin, is introduced into a mutant of the genus Escherichia, whose growth is dependent on streptomycin. Since the transformant obtained by such process harbors a plasmid comprising the chromosomal DNA fragment of Eschericha coding for the independence of streptomycin (streptomycin independent gene), the growth of the newly constructed microorganism is independent of streptomycin, i.e. the microorganism can grow in a culture medium free from streptomycin. When, however the plasmid is eliminated from the cell, the microorganism becomes dependent on streptomycin and cannot grow in a culture medium containing no streptomycin. Therefore, the instability of the characteristics of the plasmid-containing microorganism caused by the loss of the plasmid can be suppressed even without addition of antibiotic(s) to the culture medium. Therefore, the method of this invention can advantageously be applied for the commercial fermentation process.

As reported by Newcombe et al (Genetics *35* 603 (1950)), the streptomycin dependent mutant cannot grow in a culture medium without streptomycin. These mutants are sometimes dependent on paromomycin, kanamycin or ethanol (Genetics *67* 19—38 (1971)), and therefore the streptomycin-dependent-mutants of this invention include those dependent on paromomycin, kanamycin or ethanol together with the streptomycin-independance.

In order to obtain the gene responsible for the streptomycin-independence from a microorganism, whose growth is independent of streptomycin, chromosomal DNA is extracted by conventional means and treated with a restriction endonuclease.

As the vector DNA, any plasmid or phage which can propagate in microbial cells of Escherichia can be employed.

No specific method is required for inserting the chromsomal DNA fragment into the vector. The hybrid plasmid thus obtained can be incorporated into a microorganism of the genus Escherichia by conventional transformation techniques, although the efficiency may differ among these techniques.

Transformants are easily selected since these colonies can grow on a medium free from streptomycin and have a marker, such as ampicillin resistance, in the vector used.

When the plasmid in a thus obtained transformant carries the genetic information responsible for the fermentative production together with the streptomycin independent gene, the transformant apparently shows a high productivity during the fermentation, because the characteristics of the microorganisms controlled by the plasmid are stably maintained in the medium without streptomycin.

The invention is further described in the following examples.

The strains of Eschericha used in the process described were Escherichia coli K-12 (ATCC 10798, a publically available strain included in the "Catalogue of Strains I of American Type Culture Collection, 13th edition (1978)" Parent strain);

Strain FERM-P 4949, deposited on April 25, 1979 at Fermentation Research Institute=NRRL-B-12111, deposited on March 7, 1980 at Agricultural Research Culture Collection;

strain FERM-P 4987, deposited on May 21, 1979 at FERM=NRRL B-12112, deposited on March 7, 1980 at NRRL;

strain FERM-P 4898=NRRL B-12097, deposited on March 23, 1979 at FERM and February 11, 1980 at NRRL;

FERM-P 4899=NRRL B-12098, deposited on March 23, 1979 at FERM and February 11, 1980 at NRRL;

FERM-P 4900=NRRL B-12099, deposited on March 23, 1979 at FERM and February 11, 1980 at NRRL;

FERM-P 4901=NRRL B-12100, deposited on March 23, 1979 at FERM and February 11, 1980 at NRRL.

A threonine-producing microorganism harboring a plasmid inserted with two different chromosomal regions, one of them relating to threonine production and the other specifying the streptomycin independence, was obtained using streptomycin dependent strain No. 4-D (pro⁻, thiamin⁻, ile⁻, met⁻ AHV (α-amino-β-hydroxy-valeric acid)ʳ, (streptomycin)ᵈ) which is a derivative of Escherichia coli K-12, Escherichia coli AJ 11335 (FERM-P 4901, NRRL B-12100) harboring a plasmid inserted with a chromosomal region relating to theonine production (hereinafter the plasmid is referred to as "threonine-plasmid"), and Escherichia coli AJ 11346 by the following procedure:

(1) Preparation of escherichia coli AJ 11335.

(a) Preparation of chromosomal DNA possessing genetic information for high L-threonine-productivity.

Strain AJ 11332 (FERM-P 4898 NRRL B-12097) (pro⁻, thiamine⁻, ile⁻, met⁻, AHVʳ) was cultured at 37°C for 3 hours with shaking in 1l of L-medium containing 1% peptone, 0.5 % yeast extract, 0.1% glucose and 0.5% NaCl, and adjusted to pH 7.2, and bacterial cells in exponential growth phase were collected. Chromosomal DNA was extracted by the conventional phenol-method, and 5.3 mg of purified DNA were obtained.

(b) Preparation of vector DNA.

For the purpose of cloning of the gene controlling the high productivity for L-threonine (threonine operon containing mutation point of AHV resistance), DNA of plasmid pBR 322, a vector containing both genes for ampicillin and tetracycline resistance as markers, was prepared as follows.

A strain of Escherichia coli K-12 harboring the plasmid pBR 322 was incubated at 37°C in 1l of a glucose-"casamino acid"—inorganic salts medium added with 170 µg/ml of chloramphenicol. The glucose- "casamino acid"-inorganic salts medium contains 2 g glucose, 1 g NH₄Cl, 6 g Na₂HPO₄, 3 g KH₂PO₄, 5 g NaCl, 0.1 g MgSO₄· 7H₂O, 0.015 g CaCl₂· 2H₂O, 20 g "casmino acid", 0.05 g L-tryptophan, 0.05 g thymine and 100 µg thiamine · HCl, per liter, and the pH was adjusted to 7.2. By this process, the plasmid DNA was increased and accumulated abundantly in the bacertial cells.

After 16 hours of incubation, cells were harvested and then lysed by treatment with lysozyme and SDS. The lysate was centrifuged at 30,000 xg for 1 hour to obtain a supernatant. After concentrating the supernatant, 580 µg of the plasmid DNA was obtained by fractionation using cesium chloride-ethidium bromide equilibrium density gradient centrifugation.

(c) Insertion of chromosomal DNA fragment into the vector.

Each 10 µg of the chromosomal DNA and the vector DNA was treated with restriction endonuclease Hind III at 37°C for 1 hour to cleave the DNA chains, and then heated at 65°C for 5 minutes, respectively.

The digested chromosomal DNA solution and vector DNA solution were mixed and subjected to the ligation reaction of DNA fragments by the T₄ phage DNA ligase in the presence of ATP and dithiothreitol at 10°C for 24 hours. The reaction mixture was then heated at 65°C for 5 minutes and twice the volume of ethanol was added to it. The thus precipitated recombinant DNA was recovered.

(d) Genetic transformation with the hybrid plasmid harboring the genetic information for high threonine productivity.

An L-threonine-requiring strain of Escherichia coli No. 255, which was derived from threonine-producing strain AJ 11332 by N - methyl - N' - nitro - N - nitrosoguanidine mutagenesis, was cultured in 10 ml of L-medium at 37°C with shaking.

Cells in the exponential growth phase were harvested, and suspended in 0.1M MgCl₂ solution and then in 0.1M CaCl₂ solution in an ice-bath, whereby, "competent" cells having the ability of DNA uptake were prepared.

Into the competent cells suspension, the DNA obtained in step (c), which contains the hybrid plasmid DNA, was added. The suspension was kept in an ice-bath for 30 minutes, then heated at 42°C for 2 minutes and again allowed to stand in an ice-bath for 30 minutes. The cells, thus being incorporated with the DNA, were inoculated into L-medium and the medium was shaken at 37°C for 2 hours, whereby the transformation reaction was completed. The cells were collected, washed, and resuspended. A small portion of the cell suspension was spread on an agar plate containing 2g glucose, 1 g (NH₄)₂SO₄, 7g K₂HPO₄, 2gKH₂PO₄, 0.1g MgSO₄ · 7H₂O, 0.5 g sodium citrate · 2H₂O, 20 mg ampicillin, 100 mg L-proline, 100 mg L-isoleucine, 100 mg L-methionine, and 1 mg thiamine · HCl, and 2g agar, per liter, (pH was adjusted to 7.2). The plate was incubated at 37°C. After 3 days of incubation, 21 colonies appeared on the plate. All of the colonies were picked up, purified, and isolated.

Each of the transformants thus obtained did not require L-threonine and was resistant to ampicillin, which was apparently different in character from strain No. 255 employed as the recipient.

Thus a threonine-producing strain AJ 11334 (FERM-P 4900, NRRL B-12099) was obtained as the transformant harboring the threonine-plasmid.

The threonine-plasmid in AJ 11334 was isolated by a method similar to step (b), and incorporated into the parent strain AJ 11332 by a transformation technique analogous to that shown in step (d). The transformant AJ 11335 (FERM-P 4901, NRRL B-12100) was selected as an ampicillin resistance colony.

(2) Preparation of threonine-plasmid and plasmid including streptomycin independence-gene.

The DNA of the threonine-plasmid and the plasmid inserted with the streptomycin independence-gene were extracted from AJ 11335 and AJ 11346, respectively, by the following method. The strains harboring the plasmids were cultivated, cells were harvested and lysed by treatment with lysozyme and SDS. Then, the lysate was subjected to ultra-centrifugation (30.000×g) for one hour). The supernatant was then concentrated, and finally the plasmid DNA was obtained by the cesium chloride-ethidium bromide equilibrium density gradient centrifugation method.

(3) *In vitro* DNA recombination

The plasmid DNAs obtained in step (2) were mixed and treated with restriction endonuclease Bam HI at 37°C for two hours to cleave the DNA chains, and then heated at 65°C for 5 minutes. The cleaved DNA chains were recombined by the action of DNA ligase in the presence of ATP. By adding twice the volume of ethanol, the hyrbid DNAs were precipitated.

(4) Transformation with the plasmid DNA inserted with both threonine-production-gene and streptomycin independence-gene.

The streptomycin dependent strain No. 4-D derived from E. coli K-12 was cultured at 37°C with shaking in 10 ml of L-medium added with 100 μg/ml of streptomycin. In the middle stage of the exponential growth phase, cells were harvested and suspended in 0.1M $MgCl_2$ and 0.1 M $CaCl_2$ solutions in the stated order, whereby "competent" cells having the ability of DNA uptake were prepared. The competent cells were subjected to the transformation reaction using the DNA preparation obtained in step (2), the cells were then spread onto a plate of L-medium containing 20 μg/ml ampicillin, and cultured at 37°C. After 2 days of the cultivation, 1000 colonies appeared on the plate, and the colonies were replicated onto a plate of minimum medium on the whole surface of which a threonine requiring mutant cells of Escherichia coli K-12 had been spread.

The transformant colonies which excrete threonine into the plate during the cultivation were screened by detecting growth halos of the threonine-requiring mutant cells around each colony. As the results, one colony was recognized as a remarkable threonine-producer, and picked up and purified.

The strain AJ 11354 (FERM-P 4987, NRRL B-12112) thus obtained was independent of streptoymcin and capable of producing a large amount of threonine.

(5) Production of L-threonine by the novel threonine producing strain.

Table 1 shows the experimental results of the fermentative production of L-threonine using strain AJ 11354.

The cultivation medium contained 3g/dl glucose, 1g/dl $(NH_4)_2SO_4$, 0.1 g/dl $KH_2PO_4$, 0.1g/dl $MgSO_4 \cdot 7H_2O$, 10mg/dl $FeSO_4 \cdot 7H_2O$, 10mg/dl $MnSO_4 \cdot 4H_2O$, 1mg/l thiamine-HCl, 450 mg/l L-proline, 100mg/l L-isoleucine, 100mg/l L-methionine, 100mg/dl L-aspartic acid, 4g/dl $CaCO_3$, and was adjusted to pH 7.0 with KOH. Twenty ml portions of the medium were put into 500ml flasks, inoculated with AJ11334, and held for 68 hours at 30°C with shaking.

For comparison, AJ 11335 was cultured by the same manner.

TABLE 1

| Microorganism used | L-threonine produced (g/dl) |
|---|---|
| AJ 11354 | 1.21 |
| AJ 11335 | 1.12 |

Threonine was determined by a microbiological assay.

In the culture liquid of AJ 11335, there were about 20% of the cells which had lost their plasmid, while, in the culture liquid of AJ 11354, no plasmid-free cell was found.

**Claims**

1. A method for stabilizing the characteristics of a transformant microorganism of the genus Escherichia harboring a plasmid, characterized in that a plasmid obtained from a microorganism of the genus Escherichia, said plasmid being inserted with a chromosomal DNA fragment controlling the independence of streptomycin, is introduced into a mutant of the genus Escherichia, whose growth is dependent on streptomycin.

2. A method according to claim 1, wherein said plasmid is further inserted with chromosomal DNA fragment obtained from a microorganism of the genus Escherichia and relating to threonine production.

3. A method according to claim 1, wherein said mutant belongs to the species Escherichia coli.

4. A method according to claim 1, wherein said plasmid is obtained from a microorganism of the species Escherichia coli.

5. A method according to claim 1, wherein said plasmid is pBR 322.

6. A transformant obtained by introducing a plasmid obtained from a microorganism of the genus Escherichia, said plasmid being inserted with a chromosomal DNA fragment controlling the independence of streptomycin, into a mutant of the genus Escherichia whose growth is dependent on streptomycin.

7. The transformant of claim 6, in which the mutant belongs to the species Escherichia coli.

## Patentansprüche

1. Verfahren zum Stabilisieren der Eigenschaften eines transformierten Mikroorganismus des Genus Escherichia, der ein Plasmid enthält, dadurch gekennzeichnet, daß in eine Mutante des Genus Escherichia, deren Wachstum Streptomycin-abhängig ist, ein aus einem Mikroorganismus des Genus Escherichia erhaltenes Plasmid eingeführt wird, in welches das chromosomale DNA-Fragment, welches die Streptomycin-Unabhängigkeit kontrolliert, eingefügt worden ist.

2. Verfahren nach Anspruch 1, bei dem in das Plasmid außerdem das die Threonin-Produktion bestimmende chromosomale DNA-Fragment eingefügt worden ist, das aus einem Mikroorganismus des Genus Escherichia erhalten worden ist.

3. Verfahren nach Anspruch 1, bei dem die Mutante der Spezies Escherichia coli angehört.

4. Verfahren nach Anspruch 1, bei dem das Plasmid aus einem Mikroorganismus der Spezies Escherichia coli erhalten worden ist.

5. Verfahren nach Anspruch 1, bei dem das Plasmid pBR 322 ist.

6. Transformant, erhalten durch Einführen eines aus einem Mikroorganismus des Genus Escherichia erhaltenen Plasmids, in welches das die Streptomycin-Unabhängigkeit bestimmende chromosomale DNA-Fragment eingefügt worden ist, in eine Mutante des Genus Escherichia, deren Wachstum Streptomycin-abhängig ist.

7. Transformant nach Anspruch 6, wobei die Mutante der Spezies Escherichia coli angehört.

## Revendications

1. Un procédé pour stabiliser les caractéristiques d'un micro-organisme transformé du genre Escherichia contenant un plasmide, caractérisé en ce que l'on introduit dans un mutant du genre Escherichia dont la croissance dépend de la streptomycine un plasmide obtenu à partir d'un micro-organisme du genre Escherichia, ledit plasmide contenant un fragment d'ADN chromosomique commandant l'indépendance vis-à-vis de la streptomycine.

2. Un procédé selon la revendication 1, dans lequel ledit plasmide contient encore un fragment d'ADN chromosomique obtenu à partir d'un micro-organisme du genre Escherichia et concernant la production de thréonine.

3. Un procédé selon la revendication 1, dans lequel ledit mutant appartient à l'espèce Escherichia coli.

4. Un procédé selon la revendication 1, dans lequel ledit plasmide est obtenu à partir d'un micro-organisme de l'espèce Escherichia coli.

5. Un procédé selon la revendication 1, dans lequel ledit plasmide est pBR 322.

6. Un transformé obtenu par introduction, dans un mutant du genre Escherichia dont la croissance dépend de la streptomycine, d'un plasmide obtenu à partir d'un micro-organisme du genre Escherichia, ledit plasmide contenant un fragment d'ADN chromosomique commandant l'indépendance vis-à-vis de la streptomycine.

7. Le transformé selon la revendication 6, dans lequel le mutant appartient à l'espèce Escherichia coli.